# EUROPEAN PATENT SPECIFICATION

(11) **EP 3 058 330 B1**
(45) Date of publication and mention of the grant of the patent: **05.05.2021**
(21) Application number: 14806413.2
(22) Date of filing: 15.10.2014
(51) Int. Cl.: G01M 13/00, G01N 21/95, G01M 13/005, G01N 33/44

(54) **TEST ASSEMBLY FOR O-RINGS AND METHOD FOR INSPECTING O-RINGS BY MEANS OF THE ASSEMBLY ITSELF**
PRÜFANORDNUNG FÜR O-RINGE UND VERFAHREN ZUR INSPEKTION VON O-RINGEN ANHAND DER ANORDNUNG
ENSEMBLE ESSAI POUR JOINTS TORIQUES ET PROCÉDÉ D'INSPECTION DE JOINTS TORIQUES AU MOYEN DE L'ENSEMBLE LUI-MÊME

(30) Priority: 15.10.2013 IT MI20131709
(43) Date of publication of application: 24.08.2016
(73) Proprietor: UTPVision S.r.l., 24061 Albano Sant'Alessandro (BG) (IT)
(72) Inventor: FINAZZI, Roberto, I-24060 Bolgare (IT)
(74) Representative: De Bortoli, Tiziano
(86) International application number: PCT/IB2014/065328
(87) International publication number: WO 2015/056186

(56) References cited:
- EP-A1- 1 271 123
- EP-A2- 0 384 002
- DE-A1- 4 114 728

## Description

### FIELD OF THE INVENTION

The present invention relates to a test assembly for O-rings and to a method for inspecting O-rings by means of the assembly itself.

### PRIOR ART

As known, O-rings are made of elastomeric material and used as gaskets or seals to determine a sealing between mechanical members to which they are associated. O-rings have a circular section and are made in different diameters and of different materials according to the application of use. Their extremely low production costs, good performance and flexibility of use have determined their wide distribution. There are very many areas of use: from generic mechanical applications, in which the sealing between two or more members must be achieved, to particular applications, e.g. in fluid-dynamics, to achieve the sealing between mechanical members, e.g. two stretches of a pipe, in which the working pressures are extremely high.

It is equally known that the required performance of the employed O-rings must be suited to the application in which the O-rings themselves are installed. Indeed, a possible failure or weaker sealing of the O-rings with respect to the expected design values in many cases may cause the failure of the entire machinery in which they are installed, with potentially very severe consequences. In addition to the working pressures and temperatures, the type of sealing must be taken into consideration when designing and choosing the O-ring to be used. In this regard, O-rings may be used for static sealing, i.e. subject to forces of the prevalently static type; for dynamic sealing, i.e. subject to forces of the prevalently dynamic type or for mixed sealing, i.e. a combination of the two above.

Given the required performance of the O-rings and the many variables which influence such performance, it is apparent that O-ring quality and integrity is a key factor for their correct use and operation. Therefore, it is equally required to individually check the O-rings which, after their production process, could display faults.

A typical manufacturing fault consists in small cracks present on the plane of the O-ring which weaken its structure making it very prone to failures. The wide distribution and the operational requirements imposed have led sector operators to develop test methods and apparatuses to check the structural integrity of the O-rings after production before they are introduced onto the market.

A first check method includes inspecting the O-ring with a specific optical apparatus to detect the presence of cracks on the circumferential surface of the O-ring itself. Instead, a second inspection method includes the use of apparatuses provided with three rolls associated to controllable guide arms, each of which comprising a housing groove. In order to perform the inspection, a portion of the surface of the concerned O-ring is engaged in the groove of these three rolls. By means of the respective guide arms, the rolls are set at reciprocally different distances from one another, thus radially deforming the concerned O-ring in different manners. Images of the O-ring in the various deformed conditions are acquired by means of an appropriately positioned camera and the presence of possible cracks is evaluated later.

Although the reliability of the check methods in the use of O-ring models has increased, some drawbacks remain unsolved. The first method allows only to check for the presence of cracks of sizable proportions on the circumferential surface of the O-ring itself. The second test method allows to analyze the O-rings in dynamic conditions and to detect the presence of cracks which are concealed in static conditions and would otherwise go unnoticed. However, the rolls exert a radial force and are inserted in the hole defined by the diameter of the O-ring itself to deform the O-rings. Thus, such an inspection method can be applied only to O-rings having diameters compatible with the test apparatus, i.e. to O-rings with diameters greater than a given value.

EP 1 271 123 A1 discloses an apparatus for testing the quality of O-rings comprising two parallel rolling paths for receiving an O-ring and an optical device for crack testing. The two rolling paths form a testing run with a conical intake space for arranging the O-rings and a conical ejection space for transferring the individual O-rings to a discharged feed. Moreover, the two rolling paths are displaceable in the transverse direction, thereby compressing the O-ring and are driven and moved in the longitudinal direction, in order to displace the O-ring in rotation.

DE 41 14 728 A1 discloses a device for testing O-rings comprising a pair of press rollers and a roller which lies between the press rollers on one side, with a CCD camera on the opposite side. The press rollers are driven between an open position, in which the O-ring is placed therebetween, and a closed position, in which the O-ring is pressed by the press rollers. A motor 9 controls the rotation of both press rollers 7 clockwise, so as the O-ring is counter-clockwise circulated in a kind of milling action.

### SUMMARY

The Applicant realized that the known techniques partially described above do not allow to obtain an O-ring test assembly for testing different types of O-rings in reliable manner, in particular O-rings with diameters significantly different from one another. Thus, it is the main object of the present invention to make an inspection assembly for identifying cracks, which is simple and cost-effective to make, and which allows to test O-rings with different dimensional and structural features. A first specific object of the present invention is to make an inspection assembly configured to allow to analyze the two different opposite sites (defined with respect to a cross section of an O-ring). A second specific object of the present invention is to make an inspection assembly which allows to analyze O-rings continuously, and in particular O-rings with different dimensional features. A third specific object of the present invention is to make an inspection assembly with which to check all the circumferential portions which form the O-ring.

The invention thus relates to a test assembly for identifying faults in an O-ring comprising:
- a support plane, on which said O-ring rests;
- a first disc and a second disc, coplanar on said support plane and arranged in adjacent position; such discs are actuated in reciprocally independent manner and are configured to rotate on the support plane about respective axes;
- image acquisition means of said O-ring arranged at least on one side of the resting surface to acquire images of said O-ring.

The test assembly is characterized in that the discs define a test region in which the O-ring is locked and deformed between the discs themselves; the support plane is moveable and configured to carry the O-ring to a first predetermined position near an inlet section of the test region. The discs are configured to perform a first synchronized rotation in reciprocally discordant sense after which said O-ring is intercepted in said first predetermined position, dragged and locked in said test region in a first deformed configuration; the image acquisition means are configured to acquire at least one first image of the O-ring in such first deformed position. Furthermore, the discs are configured to perform at least one further synchronized rotation in reciprocally discordant sense after which the O-ring is ejected from the test region through an exit section, substantially opposite to the inlet section.

### LIST OF FIGURES:

Further features and advantages of the present invention will be more apparent in the following detailed description provided by way of non-limitative example and illustrated in the accompanying figures, in which:
- Figure 1 shows a perspective view of a test assembly according to the present invention;
- Figures from 2 to 4 show three views of a test assembly according to the present invention in three respective different configurations of use;
- Figure 5 shows a front view of a test assembly according to the present invention;
- Figures 6 and 7 show some components of a test assembly according to the present invention in two different configurations of use for O-rings of different diametrical size, respectively;
- Figures from 8 to 10 show three different steps of testing of an O-ring by means of a test assembly according to the present invention;
- Figure 11 shows an O-ring in a deformed configuration for identifying possible faults;
- Figure 12 shows some components of a test assembly according to the present invention;
- Figure 13 shows part of an inspection system for O-rings equipped with a test assembly according to the present invention.

### DETAILED DESCRIPTION

Such a description and such drawings are intended for illustrative purposes only and are consequently non-limiting; therefore, the present invention may be implemented according to other, different embodiments; furthermore, it must be considered that such figures are diagrammatic and simplified.

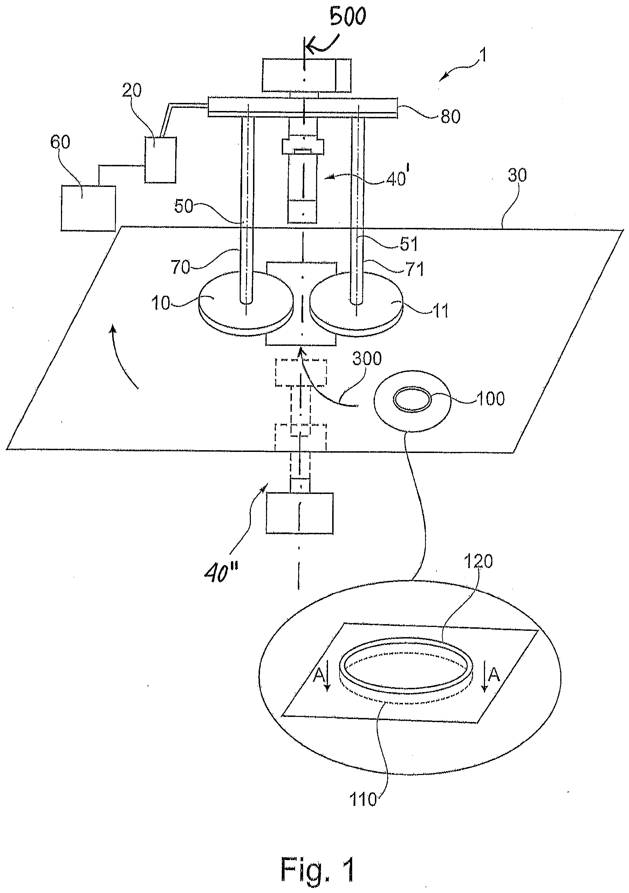

Figure 1 diagrammatically shows a test assembly 1 of an O-ring (hereinafter also indicated with the term "OR" for the sake of simplicity). In particular, the objective of the assembly 1 is to identify faults in forms of cracks deriving from the production process of the OR. The assembly 1 comprises a support plane 30 for the ORs to be tested/inspected. The assembly 1 further comprises acquisition means (40', 40") for acquiring images of the ORs. Such acquisition means are arranged at least on one side of said support plane. This means that the acquisition means can be arranged over and/or under said support plane 30, as diagrammatically shown in Figure 1. If the acquisition means are arranged under the support plane 30, this must be made of transparent material in order to allow the acquisition of the image. As specified in greater detail below, the acquisition means preferably comprise a first camera 40' and a second camera 40" arranged over and under the support plane 30, respectively, to acquire a higher number of images of the OR. As diagrammatically shown in Figure 1, the second camera 40" is indicated by a dashed line.

The assembly 1 according to the invention further comprises a first disc 10 and a second disc 11 which are adjacent and arranged coplanar on the support plane 30. The first disc 10 and the second disc 11 are independently actuated. This means that the two discs 10,11 are configured to rotate about a first axis 50 and a second axis 51, respectively, according to a rotation in either concordant or discordant sense. According to the invention, the two discs 10,11 are configured to rotate in synchronized manner and to deform the O-ring according to at least one first deformed configuration. More specifically, the two discs 10,11 deform the O-ring in the first deformed configuration by effect of a synchronized rotation in discordant sense thereof. The acquisition means are configured to acquire at least one image of the O-ring in its current first deformed configuration.

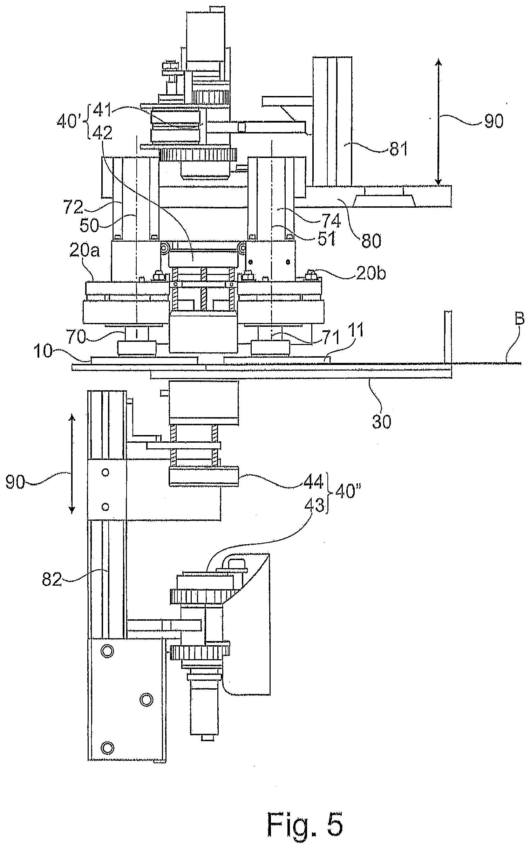

According to a preferred embodiment, the first disc 10 and the second disc 11 are fitted on the end of a first shaft 70 and of a second shaft 71, respectively. The shafts 70 and 71 are rotationally actuated, in turn, by electric motors 20a, 20b (indicated in Figure 5) that are independent so that the first shaft 70 is independent from the other 71. Preferably, stepper type motors are used. The actuation of the electric motors 20a, 20b is controlled by a control unit 60, which may also manage the actuation of the image acquisition means (assemblies 40',40" in Figure 1). Such a control unit 60 preferably comprises processing means connected to the image acquisition means to process such images.

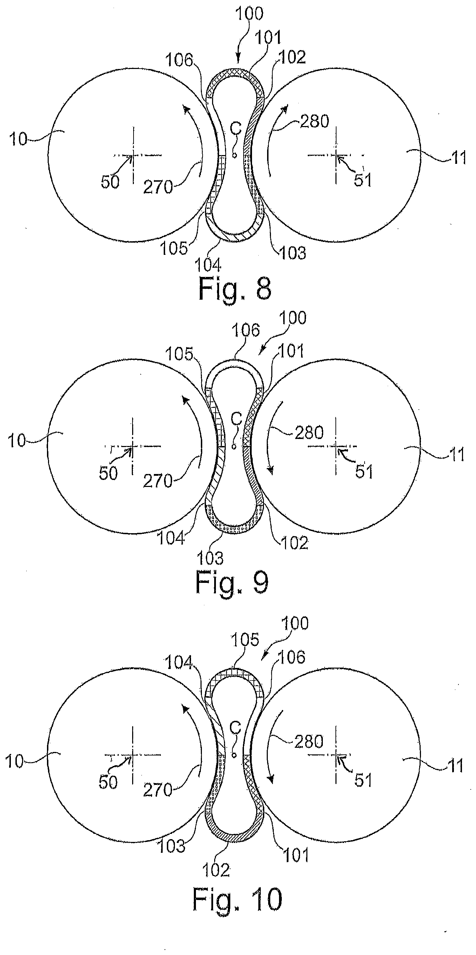

According to an embodiment, the mentioned synchronized manner comprises a first rotation manner, in which the first disc 10 rotates in discordant manner with respect to the second disc 11 (see the arrows 270 and 280 related to the first disc and to the second disc shown in Figure 8, respectively). Furthermore, a second rotation manner is provided in which the first disc 10 rotates in concordant manner with respect to said second disc 11 (see for example the arrows 270 and 280 related to the first and second disc shown in Figure 9).

Therefore, as a consequence of the above, the two discs 10,11 are configured to perform synchronized rotations in discordant sense, but also synchronized rotations in concordant sense. Such rotations synchronized in concordant sense (both discs rotate clockwise or counterclockwise), or in discordant sense (one disc rotates clockwise and the other counterclockwise) of two discs 10,11 can be obtained by virtue of the independent actuation of the discs themselves, i.e. for the presence of two reciprocally independent electric motors 20a,20b controlled by the control unit 60.

As mentioned, following a first synchronized rotation in discordant sense (first rotation manner), the first disc 10 and the second disc 11 deform the examined OR taking it to a first deformed configuration. As specified in greater detail below, according to the present invention, the first disc 10 and the second disc 11 are adapted to pass selectively from the first rotation manner to the second rotation manner to deform the OR at least in a second deformed configuration, different from the first one in order to increase the efficacy of the control.

According to the invention, the first disc 10 and the second disc 11 are configured and reciprocally positioned so as to "capture" an OR in the space region comprised between the two discs 10, 11 by means of a first synchronized rotation in discordant sense (hereinafter also indicated as "counter-rotation") of the discs themselves. Substantially, the word "counter-rotation" means the first rotation manner (discordant rotation) of the two discs 10,11 such that the first disc 10 rotates in a sense opposite to that of the second disc 11.

The two discs 10,11 are thus configured so as to deform and lock, following such a counter-rotation, the examined OR in at least one first deformed configuration. When the deformed configuration is reached, the image acquisition means acquiring one or more images of the deformed OR to allow to check the integrity of the OR, i.e. the presence of faults (cracks) in the ring itself.

In order to be captured and deformed by the two discs 10, 11, the OR to be inspected is taken to a first predetermined position, hereinafter also indicated with the expression "capture position". For such a purpose, the support plane 30 is preferably moveable to carry the OR to be inspected to a predetermined position corresponding precisely to the capture position defined above. The support plane 30 is preferably checked by means of the control unit 60.

As diagrammatically shown in Figure 1, the support plane 30 consists of a rotating plane which automatically carries the rings to be inspected to the capture position according to a circular trajectory, as indicated by reference numeral 300. Alternatively, the support plane could also be formed by a conveyor belt or equivalent means which allow to reach the capture position by means of rectilinear trajectories. Advantageously, all these configurations guarantee full automation for inspecting one or more ORs. However, the concept underlying the present invention includes deforming an OR from the outside of its perimeter and acquiring at least one image in this deformed configuration to evaluate the presence of cracks and/or production faults.

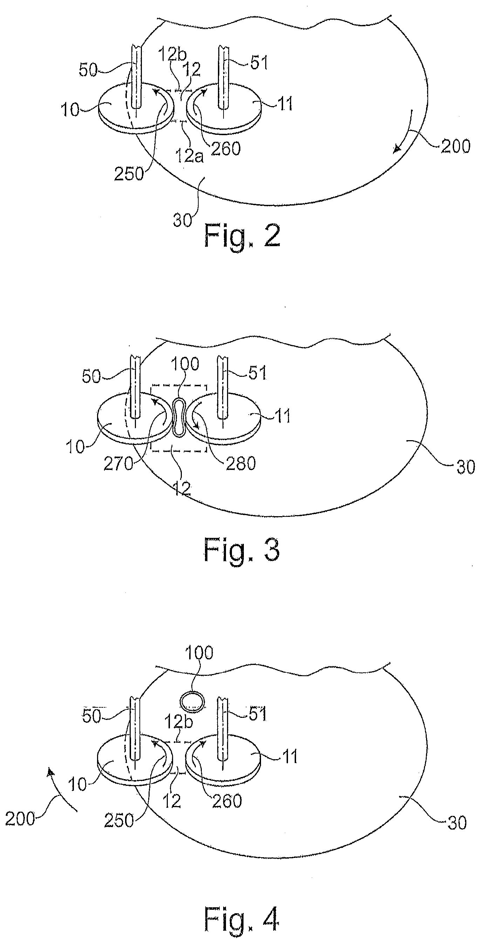

Figures from 2 to 4 diagrammatically show a possible operation of the test assembly 1 according to the present invention. Figure 2 shows the ring to be inspected (indicated by reference numeral 100) in the capture position. The space substantially comprised between the two discs 10,11 at which the OR is deformed and locked is indicated hereinafter with the expression "test region 12". The "capture position" of the OR is operatively defined at the inlet 12a of the test region 12. The OR reaches the capture position following the movement of the support plane 30.

Following a first synchronized rotation in discordant sense (first counter-rotation) of the discs 10,11 (identified by the arrows 250, 260), the OR to be inspected is intercepted by the discs 10,11 in the capture position of the discs themselves. More specifically, the discs 10,11 intercept the OR by dragging it and locking it in the test region 12 in deformed configuration.

The OR is dragged into the test region 12 through the section inlet 12a indicated above. By effect of the conformation of the discs 10,11, the OR is deformed, from the outside, in fact squeezed between the discs themselves, as diagrammatically shown in Figure 3. Substantially, the counter-rotation of two discs 10,11 is thus defined (in terms of rotation angles and speed) by the control unit 60, so that at the end of the same the OR is arranged in the test region 12, locked between the two discs 10,11 in the first deformed configuration to allow the acquisition of images by means of the acquisition means 40,40'.

In this regard, the image acquisition means (assemblies 40',40") define a main optical axis 500, which means, according to a definition widely recognized in the sector, the line which joins the centers of the spherical caps which form the lenses of the image acquisition means. The control unit 60 is configured so that at the end of the first synchronized rotation in discordant sense the center C of the OR is aligned with such an optical axis 500. In this manner, the center C of the OR is in fact in the center of the acquired image or images.

With reference to Figure 4, at the end of the image acquisition process, according to the invention, the discs 10,11 are configured to perform a further synchronized rotation in reciprocally discordant sense (second counter-rotation), following which the OR is ejected through an outlet section 12b of the test zone 12 to reach a second position, indicated hereinafter as "ejection position". The outlet section 12b is substantially opposite to the inlet section 12a with respect to the test region 12. By means of this solution, the inspection process of the O-ring occurs continuously. In particular, the entrance and the exiting of the OR into/from the test region 12 does not require any movement of the discs 10,11. Substantially, during the entrance and the exiting of the OR into/from the test region 12, the rotation axes 50,51 of the discs 10,11 remain fixed.

Figure 4 shows an OR precisely in the ejection position. Also in Figure 4, the arrows 250, 260 show the contrary rotation senses of the two discs 10, 11 in order to implement the second counter-rotation of the two discs 10,11.

After having reached the ejection position, according to the result of the inspection, the examined OR may be either rejected, in case of presence of faults, or included in a supply, in case of absence of faults. For this purpose, the ORs may be selected by means of an automatic system, comprising, for example, a pneumatic actuation.

As explained above with reference to Figure 3, the position and the configuration of the discs 10,11 is such that they act on the OR deforming it from the outside. This aspect is very advantageous because the deformation of the OR may be implemented independently from the diameter of the OR contrary to the traditional solutions.

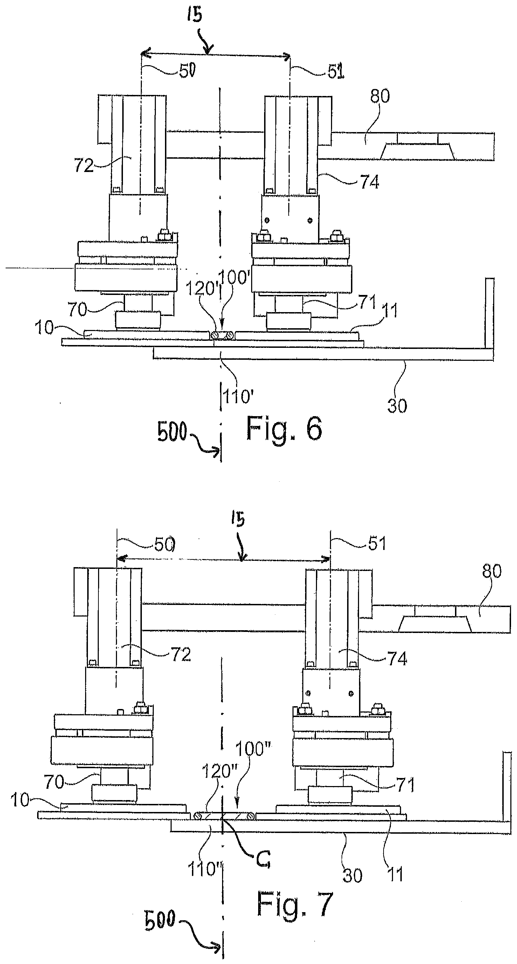

Figures 5, 6 and 7 are perspective views related to a first embodiment of an inspection assembly 1 according to the present invention. According to the invention, the assembly 1 comprises first adjustment means for adjusting the distance between the rotation axes of the discs 10,11 as a function of the diametrical extension of the ORs to be tested. In this regard, the assembly 1 preferably comprises a supporting frame which includes a crossbar 80, a first supporting arm 72 and a second supporting arm 74. The first supporting arm 72 operatively supports the first disc 10, while the second supporting arm 74 operatively supports the second disc 11. The supporting arms 72, 74 are connected in sliding manner to the crossbar 80. In this manner, the distance of the supporting arms 72, 74 and consequently the distance between the axes 50,51 of the two discs 10,11 may be advantageously adjusted as a function of the diameter of the ORs. In this regard, Figures 6 and 7 show two different configurations of use of the assembly 1, each adapted to inspect an OR of a given diameter. The configuration in Figure 6 is adapted to inspect an OR 100' of relatively small size, with respect to that of the OR 100" which can be inspected in the configuration shown in Figure 7. As apparent by comparing such figures, the distance (indicated by reference numeral 15) between the axes 50,51 of the two discs 10,11 is directly proportional to the diameter of the ORs to be tested/inspected.

Figure 5 is a front view of the assembly 1 which shows a preferred embodiment of the image acquisition means. The latter preferably comprise a first assembly 40', which includes a first camera 41 and a first illuminator 42 arranged over the support plane 30, and thus over the first disc 10 and the second disc 11. The acquisition means also comprise a second assembly 40", which includes a second camera 43 and a second illuminator 44 arranged under the support plane 30 itself in symmetric position respective to the first camera 41 and to the first illuminator 42. For such a purpose, the support plane 30 is made of transparent material. The function of the illuminators 42, 43 is to increase the light intensity on the upper portion 120 or on the lower portion of the O-ring so as to allow the cameras to acquire very sharp images.

The embodiment shown in Figure 5 is particularly advantageous because it allows to acquire images from the two sides of the OR, more specifically the upper side 120 and the lower side 110 (shown in Figure 1), in deformed configuration. Figure 5, again, shows that the assembly 1 comprises second adjustment means configured to adjust the position of the cameras and of the illuminators with respect to the support plane 30. In particular, in the illustrated case in point, a first supporting member 81, which develops vertically over the support plane 30, and a second member 82, which develops vertically under the plane 30 itself, are provided. The first camera 41 and the first illuminator 42 are slidingly associated to the first member 81, while the second camera 42 and the second illuminator 42 are slidingly associated to the second element 82. In this manner, the position of the cameras 41, 43 and of the illuminators 42, 44 can thus be adjusted in the vertical direction, indicated with reference numeral 90 in Figure 5.

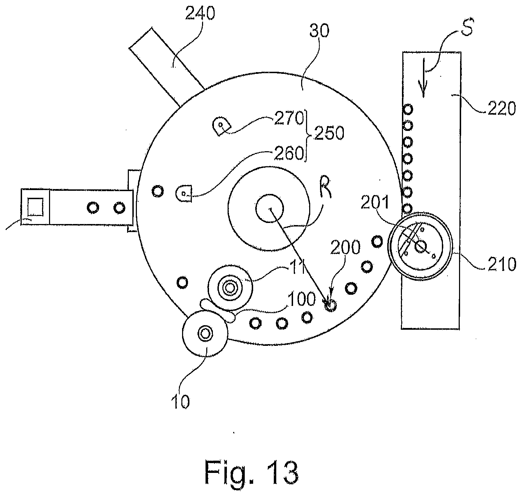

In the embodiment shown in Figures from 5 to 7 and in Figure 13, it can be observed that the plane 30 is preferably defined by a circular rotating table and that the OR rests on the plane 30 in proximity of a region near the edge of such a circular table at a predetermined radial distance R (indicated in Figure 13) from the rotation center of the rotating table. Such a distance can be evaluated between the rotation axis of the rotating table and the center C of the ring. It is worth noting that by effect of the configuration of the discs 10,11 and the deformation principle developed by the test assembly 1, the radial distance R remains substantially the same during the entire inspection process (entrance, permanence and exiting into/in/from the test region 12).

With reference to the last mentioned figures again, the two discs 10,11 are positioned corresponding to this lying configuration of the OR. Part of the disc 10 protrudes from the plane 30, while the disc 11 is at the plane 30, to define the test region 12 (not shown in Figure 5) at the edge of the plane 30 on which the OR rests. In this manner, the ORs are tangentially driven between the two discs 10,11. Anyway, it is worth noting that the operating position of the two discs 10,11 is established as a function of the radial distance R on which the ORs transit.

For the correct acquisition of images, the position of the two cameras 41, 42 will be varied indeed in vertical direction in order to obtain the most correct focusing to detect faults and cracks of any size. The focusing may be adjusted as a function of the concerned OR or the size of the cracks to be identified.

According to a preferred embodiment of the invention, following the acquisition of images related to the first deformed configuration, the two discs 10,11 perform at least one first rotation synchronized in concordant sense, after which the OR is deformed and locked in the test region 12 in a second deformed configuration different from the first deformed configuration. Preferably, after having acquired images related to the first deformed configuration, the two discs 10,11 perform a plurality of synchronized rotations in concordant sense, after each of which the OR is deformed and locked in the test region 12 to acquire respective images by means of the acquisition devices described above. Each deformed configuration obtained following one of such synchronized rotations in concordant sense is different from the others and from the first deformed configuration obtained with the synchronized rotation in discordant sense.

The control unit 60 is configured so that at the end of each synchronized rotation in concordant sense the center C of the OR is aligned with the main optical axis 500 of the imagine acquisition means. In this manner, for each deformed configuration, the center C of the OR coincides with the center of the image or images.

At the end of the acquisition process, related to the last deformed configuration which is obtained, the two discs 10,11 will indeed perform a further rotation in discordant sense in order to expel the OR from the test region 12 through the outlet section 12b, as described above.

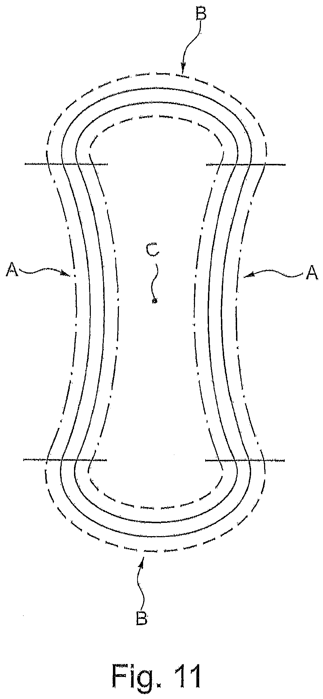

Figure 11 illustrates what inward or outward deformation and "deformed configuration" mean for the purposes of the present invention. Figure 11 shows a first deformed configuration of the OR. The discs 10,11 are not shown for the sake of clarity. As mentioned, the deformation is induced, for example by the discs 10,11, from the outside of the perimeter of the OR. The circular shape of the OR and the size of the discs 10,11 induce, in fact, two types of deformations of the portions which form the OR. In particular, the first two reciprocally symmetric portions (with respect to the center C) are deformed by traction (identified by reference A, i.e. the portions surrounded by the dashed and dotted line); and the second two reciprocally symmetric portions (again with respect to the center C) are deformed by compression (identified by reference B, i.e. the portions surrounded by the dashed lines). During a subsequent step, the discs 10,11, by means of any appropriate synchronized rotation in concordant sense, drag the OR deforming it to reach a second deformed configuration, different from the first one in which the first portions (A in Figure 11) are under compression, while the second portions (B in Figure 11) are under traction. The possibility of acquiring images of the OR in several deformed configurations translates into the possibility of inspecting an OR in more precise, accurate manner, being able to detect cracks of different type.

Figures from 8 to 10 diagrammatically show three steps of a possible embodiment of the inspection method of an O-ring, using an assembly 1 according to the present invention. In particular, such an inspection method is implemented by using an assembly 1 in the configuration shown in Figures from 5 to 7, i.e. using acquisition means capable of acquiring images from both sides of the examined OR.

In general, the method for inspecting O-rings according to the present invention comprises the steps of:
- arranging the O-ring on a support plane 30;
- arranging image acquisition means of the O-ring on at least one side of the support plane 30;
- carrying the O-ring, through the moveable support plane 30, to a capture position (predetermined position) near the inlet section 12a of the test region 12;
- implementing a first synchronized rotation in discordant sense of said discs 10,11 to intercept said O-ring in said predetermined position and to drag and lock said O-ring in said test region 12 in said first deformed configuration;
- acquiring, by means of said acquisition means at least one image of the O-ring in said first deformed configuration;
- implementing a further synchronized rotation in discordant sense of said discs 10,11 to expel said O-ring from said test region (12) through said outlet section (12b).

A preferred embodiment of the method according to the present invention further comprises the step of actuating (following the acquisition of images in said first deformed configuration and before actuating said further synchronized rotation) one or more synchronized rotations in concordant sense of said discs 10,11. Each of such synchronized rotations is actuated to deform and lock the OR in a corresponding deformed configuration. The method includes acquiring one or more images of said O-ring when each deformed configuration, obtained following a corresponding of said synchronized rotations in concordant sense of said discs 10,11, is reached.

As mentioned above, it is worth noting that the examined OR remains in the same position with respect to the plane 30 during the various deformed configurations to which it is subjected. This feature is also apparent by comparing Figures 8, 9 and 10, in which the six portions of OR assume different angular positions for each deformed configuration, however the OR remains in the same position with respect to the plane 30 for the entire test time (in this case, during the three deformed configurations it assumes). In particular, the center C of the OR lays on the plane defined by the rotation axes 50,51 of the two discs 10,11. Such a condition is obtained and maintained by effect of the independent control of the two discs 10,11 and by means of an appropriate definition in terms of accelerations and speed, of the synchronized rotation manner in concordant sense of the discs 10,11. Advantageously, in this manner, the field of view of the camera is fully exploited at the maximum available resolution because such a field of view remains in fact unchanged during the entire deformation process of the ring and the image acquisition.

The assembly 1 according to the invention comprises processing means connected to said image acquisition means and to the control unit 60. Preferably, the processing means are integrated in said control unit 60. The function of such processing means is to check for the presence of cracks/faults in the OR structure. According to a preferred embodiment, the processing means are configured to check the position of the center C of the OR with respect to an ideal position coinciding with the main optical axis 500 of the image acquisition means.

In particular, the real position of the center C of the O-ring is defined by analyzing each image acquired by the image acquisition means. Such a real position is compared with the ideal position to evaluate the possible positioning error. If such an error reaches a predetermined value, the processing means calculate new operating conditions for the rotation of the discs (speed-accelerations) in order to compensate the positioning error by means of the subsequent synchronized rotation of the two discs 10,11. The control unit 60, is connected to the processing means, will actuate the electric motors 20a,20b of the two discs 10,11 in manner corresponding to such operating conditions actuating such error compensation.

According to a further embodiment, the method according to the present invention can be adapted as a function of the size of the examined OR. For this purpose, a preliminary step of the method includes adjusting the distance between the rotation axes 50,51 of said discs 10,11, as a function of the diametrical size of the O-ring and of the desired deformation. Indeed, the diameter of the O-ring being equal, as the distance between the discs 10,11 decreases, the ring itself is further "squeezed", i.e. deformed, between the discs themselves. The distance between the rotation axes 50,51 may be adjusted either manually or automatically by means of the control unit 60.

Thus, turning back to Figures 8, 9 and 10, for the sake of better understanding, the OR is considered formed by six contiguous peripheral portions, in particular: a first portion 101, a second portion 102, a third portion 103, a fourth portion 104, a fifth portion 105 and a sixth portion 106. By effect of the rotation of the two discs 10,11, each of these six portions undergoes a deformation either inwards or outwards.

In particular, Figure 8 shows the OR in a first deformed configuration following the first synchronized rotation in discordant sense (first rotation manner of the discs 10,11). The deformation of the examined OR occurs on the support plane 30. By effect of their circular conformation, the two discs 10,11 deform the examined ring by deforming under traction the peripheral second portion 102, the third portion 103, the fifth portion 105 and the sixth portion 106 towards the center C of the OR; the peripheral first portion 101 and the fourth portion 104 are deformed under compression, i.e. outwards with respect to the center C.

When such a first deformed configuration is reached, the acquisition means 40 acquire one or more images in order to highlight the presence of cracks through the structure of the OR.

Figure 9 shows a step subsequent to that shown in Figure 8, in which the OR is in a second deformed configuration. The OR is taken to this second configuration by means of a synchronized rotation in concordant sense (i.e. passing from the first rotation manner of the discs 10,11 to their second rotation manner).

In the second deformed configuration in Figure 9, the sixth portion 106 and the third portion 103 are under compression, while the first portion 101, the second portion 102, the fourth portion 104 and the fifth portion 105 are under traction. In this second deformed configuration, one or more images of the OR are acquired to check for the presence of cracks.

Figure 10 shows a third step of the inspection method according to the present invention, in which the OR is in a third deformed configuration. As shown in Figure 9, the third deformed configuration is obtained by means of a further rotation in concordant sense of the discs 10,11 (e.g. by acting on the angular speeds of each disc). In the third deformed configuration, it is worth noting that the fifth portion 105 and the second portion 102 are under compression; while the third portion 103, the fourth portion 104, the first portion 101 and the sixth portion 106 are under traction. Also in the third deformed configuration, one or more images of the OR are acquired to check for the presence of cracks.

Thus, according to the described embodiment, each portion of OR is deformed in two different manners: by compression and by traction. At least one image of the OR is acquired in this condition of deformation to evaluate the presence of cracks.

According to an embodiment, the plurality of images acquired by means of the acquisition means in the first, second and third deformed configuration are stored in the processing means which are connected at least to the acquisition means and indeed used to check for the presence of cracks/faults. For this purpose, a graphic post-processing software program is preferably loaded and run in real time on the processing unit indicated above aimed at identifying the presence of cracks. In this manner, the O-ring may be rejected in real time immediately after having acquired the images according to the presence of possible cracks. The rotation speed of the discs 10,11 and the acquisition and storing speed of the images of the two sides of the O-ring 100 are chosen so as to be compatible with a real time control of the O-ring. Furthermore, the selectively configurable rotation axes 50,51 and the adjustable focus of the two cameras 41,43 allows to test different O-rings with reciprocally different dimensional and structural features in real time.

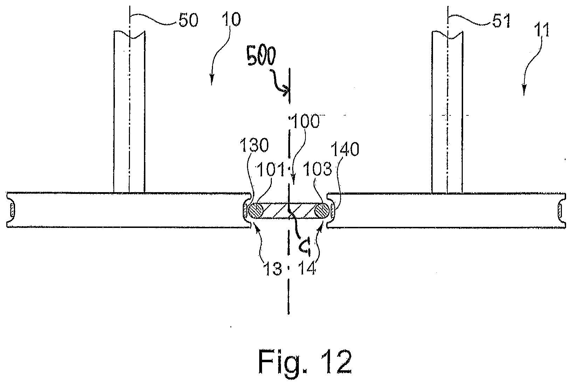

With reference to Figure 12, it is possible to view a detail of an embodiment of the discs 10,11. Such discs are respectively provided with a circumferential groove 13,14 in which opposite peripheral portions 101,103 of an O-ring 100 are respectively housed. In order to improve the deformation action produced by the discs 10,11, the grooves 13,14 are coated with a specific layer of material 130,140, preferably of non-slip material. In this manner, the sliding friction between the walls of the grooves 13,14 and the corresponding outer peripheral portions 101,103 of the O-ring 100 are increased, so that the latter can be taken to the various deformed configurations more effectively.

Figure 13 shows part of an OR inspection system equipped with a test assembly according to the present invention. In the embodiment shown in the figure, the inspection system further comprises an OR loading module 220. Such a module supplies the ORs to be tested, in particular is configured to expel them according to a direction S. The force applied on the OR by the ejection causes it to impact against a septum 210. The OR stops on the rotating plane in this manner. The rotating plane 30 (which in the embodiment shown in the figure rotates counterclockwise) takes the ORs to the discs 10,11. The ORs 200 to be inspected are taken onto the rotating plane 30 at a peripheral edge thereof. The OR 100 under test is inspected according to the methods described above. It is worth noting that the rotation of the table 30 is synchronized with the rotation of the discs 10,11 to avoid the superimposition of the ORs under test 100 with the ORs 200 to be inspected. It is worth noting that the ORs 200 have different size. The distance between the rotation axes 50,51 is adjusted according to the size. When the inspection of an OR ends, the synchronized rotation of the discs 10,11 releases it from the test zone identified between the discs themselves (see Figure 4). The OR is either rejected or kept according to whether a crack is present or not. For this purpose, the software program loaded and run on the control unit of the test assembly by the storing and processing means, respectively, allows to identify the presence of cracks by analyzing the captured image and furthermore to control the corresponding rejecting or keeping of the OR. According to an embodiment, an OR selection assembly 250 is thus provided. Such an assembly comprises a first actuator 260 (preferably a source of compressed air) to take the OR free from cracks into a first specific container 230; and a second actuator 270 (preferably a source of compressed air) to take the OR with cracks into a second specific container 240. The first actuator 260 and the second actuator 270 are selectively controlled by the control unit 60 according to the analysis of the captured image aimed at detecting the presence of cracks.

## Claims

1. A test assembly (1) for inspecting an O-ring (100), comprising:
- a support plane (30), on which said O-ring rests;
- a first disc (10) and a second disc (11) coplanar on said support plane (30) and arranged in adjacent position, being configured to rotate on said support plane (30) about respective axes (50, 51);
- image acquisition means (40',40") of said O-ring arranged at least on one side of said support plane (30) to acquire images of said O-ring,
**characterized in that** said discs (10, 11) are actuated in reciprocally independent manner and define a test region (12) at which said O-ring is locked and deformed between said discs (10,11), said support plane (30) being moveable and configured to carry said O-ring to the first predetermined position near an inlet section (12a) of said test region (12),
wherein said discs (10, 11) are configured to perform a first synchronized rotation in reciprocally opposite sense after which said O-ring is intercepted in said first predetermined position, dragged and locked in said test region (12) in a first deformed configuration, said image acquisition means (40',40") being configured to acquire at least one first image of said O-ring in said first deformed position,
and wherein said discs (10,11) are configured to perform at least one further synchronized rotation in reciprocally opposite sense after which said O-ring is ejected from said test region (12) through an exit section (12b), substantially opposite to said inlet section (12a).

2. An assembly (1) according to claim 1, wherein after said first synchronized rotation in reciprocally opposite sense of said discs (10,11), the center (C) of said O-ring lays on a plane defined by the rotation axes (50,51) of said discs (10,11).

3. An assembly (1) according to claim 1 or 2, wherein said discs (10,11) are configured to perform, between said first rotation and said second rotation synchronized in opposite sense, at least one synchronized rotation in the same sense after which said O-ring is deformed and locked in said test region (12) in a second deformed configuration, different from said first deformed configuration, said image acquisition means (40',40") being configured to acquire at least one first image of said O-ring in said second deformed configuration.

4. An assembly (1) according to claim 1 or 2, wherein said discs (10,11) are configured to perform, between said first rotation and said second rotation synchronized in opposite sense, a plurality of synchronized rotations in the same sense after each of which said O-ring is deformed and locked in said test region (12) in a deformed configuration, different from said first deformed configuration, said image acquisition means (40',40") being configured to acquire at least one first image of said O-ring in each deformed configuration.

5. An assembly (1) according to any one of the preceding claims, wherein said first assembly comprises a first electric motor (20a) and a second electric motor (20b) for actuating said first disc (10) and said second disc (11), respectively.

6. An assembly (1) according to any one of the preceding claims, wherein said assembly comprises first adjustment means for adjusting the distance between said rotation axes (50,51) of said discs (10,11) as a function of the diametrical extension of said O-ring and the desired deformation.

7. An assembly (1) according to claim 6, wherein said assembly (1) comprises a supporting frame, which includes a crossbar (80), a first supporting arm (72) and a second supporting arm (74) to support said first disc (10) and said second disc (11), respectively, said arms (72, 74) being connected in sliding manner on said crossbar to adjust said distance between said rotation axes (50,51) of said discs (10,11).

8. An assembly (1) according to any one of the proceeding claims, wherein said first disc (10) and said second disc (11) comprise a circumferential groove (13,14) adapted to house and drag said O-ring, respectively.

9. An assembly (1) according to any one of the preceding claims, wherein said support plane (30) is made of transparent material and wherein said acquisition means comprise a first camera (41) and a second camera (42) arranged in symmetric position with respect to said support plane (30).

10. An assembly (1) according to claim 9, comprising a first illuminator (42) arranged between said support plane (30) and said first camera (41) and a second illuminator (43) arranged between said support plane (30) and said second camera (42).

11. An assembly (1) according to any one of the preceding claims, wherein said assembly (1) comprises a control unit (60) which controls said electric motors (20a,20b).

12. An assembly (1) according to any one of the claims from 1 to 11, wherein said assembly (1) comprises processing means connected to said acquisition means (40',40") and to a control unit (60) which controls said electric motors (20a,20b) for rotating said discs (10,11), said processing means being configured to check the position of the center (C) of said O-ring with respect to an ideal position coinciding with a main optical axis (500) defined by said image acquisition means (40',40"), said real position being evaluated by analyzing each image acquired from said image acquisition means (40',40").

13. An assembly (1) according to claim 12, wherein said processing means are configured to compare said ideal position in order to establish a positioning error, said processing means calculating the corresponding operating conditions for the subsequent synchronized rotation of the said discs (10,11) if said positioning error exceeds a predetermined value, said control unit (60) actuating said electric motors (20a,20b) as a function of said corresponding operating conditions.

14. A system for inspecting O-rings, **characterized in that** it comprises a test assembly (1) according to any one of the claims from 1 to 11.

15. A method for inspecting an O-ring by means of an inspection assembly (1) according to any one of the claims from 1 to 14, wherein said method comprises the steps of:
- arranging said O-ring on said support plane (30);
- arranging image acquisition means of the O-ring on at least one side of said support plane (30);
- activating said support plane (30) to carry said O-ring to said predetermined position near said inlet section (12a) of said test region (12);
- implementing a first synchronized rotation in opposite sense of said discs (10,11) to intercept said O-ring in said predetermined position and to drag and lock said O-ring in said test region (12) in said first disturbed configuration;
- activating said acquisition means to acquire at least one image of the O-ring in said first deformed configuration;
- implementing a further synchronized rotation in opposite sense of said discs (10,11) to expel said O-ring from said test region (12) through said outlet section (12b).

16. A method according to claim 15, wherein said method comprises the step of actuating, after the acquisition of images in said first deformed configuration and before the actuation of said further synchronized rotation, one or more synchronized rotations in the same sense of said discs (10,11), each of which to deform and lock the O-ring in a corresponding deformed configuration, said method further comprising the step of actuating said acquisition means when each deformed configuration is reached.

## Patentansprüche

1. Testanordnung (1) zur Inspektion eines O-Rings (100), bestehend aus:
- eine Trägerebene (30), auf der der besagte O-Ring aufliegt;
- eine erste Scheibe (10) und eine zweite Scheibe (11), die koplanar auf der Trägerebene (30) und in benachbarter Position angeordnet sind, wobei sie so konfiguriert sind, dass sie sich auf der Trägerebene (30) um jeweilige Achsen (50, 51) drehen;
- Bildaufnahmemittel (40', 40") des O-Rings, die mindestens auf einer Seite der Trägerebene (30) angeordnet sind, um Bilder des O-Rings aufzunehmen,
**dadurch gekennzeichnet, dass** die Scheiben (10, 11) in wechselseitig unabhängiger Weise betätigt werden und einen Testbereich (12) definieren, in dem der O-Ring zwischen den Scheiben (10, 11) verriegelt und verformt wird, wobei die Stützebene (30) beweglich und so konfiguriert ist, dass sie den O-Ring in die erste vorbestimmte Position nahe einem Einlassabschnitt (12a) des Testbereichs (12) mitnimmt,
wobei die Scheiben (10, 21) so konfiguriert sind, dass sie eine erste synchronisierte Drehung in wechselseitig entgegengesetzter Richtung durchführen, wonach der O-Ring in der ersten vorbestimmten Position abgefangen, in den Testbereich (12) in einer ersten verformten Konfiguration gezogen und verriegelt wird, wobei die Bildaufnahmemittel (40', 40") so konfiguriert sind, dass sie mindestens ein erstes Bild des O-Rings in der ersten verformten Position aufnehmen,
und wobei die Scheiben (10, 11) so konfiguriert sind, dass sie mindestens eine weitere synchronisierte Drehung in wechselseitig entgegengesetzter Richtung durchführen, wonach der O-Ring durch einen Austrittsabschnitt (12b), der im Wesentlichen dem Einlassabschnitt (12a) gegenüberliegt, aus dem Testbereich (12) ausgeworfen wird.

2. Anordnung (1) nach Anspruch 1, wobei nach der ersten synchronisierten Drehung der Scheiben (10, 11) in wechselseitig entgegengesetzter Richtung der Mittelpunkt (C) des O-Rings auf einer Ebene liegt, die durch die Drehachsen (50, 51) der Scheiben (10, 11) definiert ist.

3. Anordnung (1) nach Anspruch 1 oder 2, wobei die Scheiben (10, 11) so konfiguriert sind, dass sie zwischen der ersten Drehung und der zweiten Drehung, die in entgegengesetzter Richtung synchronisiert sind, mindestens eine synchronisierte Drehung in derselben Richtung ausführen, nach der der O-Ring verformt und in dem Testbereich (12) in einer zweiten verformten Konfiguration, die sich von der ersten verformten Konfiguration unterscheidet, verriegelt wird, wobei die Bildaufnahmemittel (40', 40") so konfiguriert sind, dass sie mindestens ein erstes Bild des O-Rings in der zweiten verformten Konfiguration aufnehmen.

4. Anordnung (1) nach Anspruch 1 oder 2, wobei die Scheiben (10, 11) so konfiguriert sind, dass sie zwischen der ersten Drehung und der zweiten Drehung, die in entgegengesetzter Richtung synchronisiert sind, eine Vielzahl von synchronisierten Drehungen in derselben Richtung ausführen, nach denen der O-Ring jeweils verformt und in dem Testbereich (1) in einer verformten Konfiguration, die sich von der ersten verformten Konfiguration unterscheidet, verriegelt wird, wobei die Bildaufnahmemittel (40', 40") so konfiguriert sind, dass sie mindestens ein erstes Bild des O-Rings in jeder verformten Konfiguration aufnehmen.

5. Anordnung (1) nach einem der vorstehenden Ansprüche, wobei die erste Anordnung einen ersten Elektromotor (20a) und einen zweiten Elektromotor (20b) zum Betätigen der ersten Scheibe (10) bzw. der zweiten Scheibe (11) umfasst.

6. Anordnung (1) nach einem der vorstehenden Ansprüche, wobei die Anordnung erste Einstellmittel zum Einstellen des Abstands zwischen den Drehachsen (50, 51) der Scheiben (10, 11) als Funktion der diametralen Ausdehnung des O-Rings und der gewünschten Verformung umfasst.

7. Anordnung (1) nach Anspruch 6, wobei die Anordnung (1) einen Tragrahmen umfasst, der eine Querstrebe (80), einen ersten Tragarm (72) und einen zweiten Tragarm (74) zum Tragen der ersten Scheibe (10) bzw. der zweiten Scheibe (11) einschließt, wobei die Arme (72, 74) gleitend auf der Querstrebe verbunden sind, um den Abstand zwischen den Drehachsen (50, 51) der Scheiben (10, 11) einzustellen.

8. Anordnung (1) nach einem der vorstehenden Ansprüche, wobei die erste Scheibe (10) und die zweite Scheibe (11) eine Umfangsnut (13, 14) umfassen, die zur Aufnahme bzw. zum Ziehen des O-Rings ausgelegt ist.

9. Anordnung (1) nach einem der vorstehenden Ansprüche, wobei die Trägerebene (30) aus transparentem Material hergestellt ist und wobei die Aufnahmemittel eine erste Kamera (41) und eine zweite Kamera (42) umfassen, die in symmetrischer Position in Bezug auf die Trägerebene (30) angeordnet sind.

10. Anordnung (1) nach Anspruch 9, umfassend eine erste Beleuchtungseinrichtung (42), die zwischen der Trägerebene (30) und der ersten Kamera (41) angeordnet ist, und eine zweite Beleuchtungseinrichtung (43), die zwischen der Trägerebene (30) und der zweiten Kamera (42) angeordnet ist.

11. Anordnung (1) nach einem der vorstehenden Ansprüche, wobei die Anordnung (1) eine Steuereinheit (60) umfasst, die die Elektromotoren (20a, 20b) steuert.

12. Anordnung (1) nach einem der Ansprüche 1 bis 11, wobei die Anordnung (1) Verarbeitungsmittel umfasst, die mit dem Aufnahmemittel (40', 40") und mit einer Steuereinheit (60) verbunden ist, die die Elektromotoren (20a, 20b) zum Drehen der Scheiben (10, 11) steuert, wobei die Verarbeitungsmittel konfiguriert sind, um die Position des Zentrums (C) des O-Rings in Bezug auf eine ideale Position zu prüfen, die mit einer optischen Hauptachse (500) zusammenfällt, die durch das Bildaufnahmemittel (40', 40") definiert ist, wobei die reale Position durch Analysieren jedes von dem Bildaufnahmemittel (40', 40") aufgenommenen Bildes ausgewertet wird.

13. Anordnung (1) nach Anspruch 12, wobei die Verarbeitungsmittel konfiguriert sind, um die ideale Position vergleichen, um einen Positionierungsfehler festzustellen, wobei die Verarbeitungsmittel die entsprechenden Betriebsbedingungen für die anschließende synchronisierte Drehung der Scheiben (10, 11) berechnen, wenn der Positionierungsfehler einen vorbestimmten Wert überschreitet, wobei die Steuereinheit (60) die Elektromotoren (20a, 20b) in Abhängigkeit von den entsprechenden Betriebsbedingungen betätigt.

14. System zur Inspektion von O-Ringen, **dadurch gekennzeichnet, dass** es eine Testanordnung (1) nach einem der Ansprüche von 1 bis 11 umfasst.

15. Verfahren zur Inspektion eines O-Rings mittels einer Inspektionsanordnung (1) nach einem der Ansprüche von 1 bis 14, wobei das Verfahren die Schritte umfasst von:
- Anordnen des O-Rings auf der Trägerebene (30);
- Anordnen von Bildaufnahmemitteln des O-Rings auf mindestens einer Seite der Trägerebene (30);
- Aktivieren der Trägerebene (30), um den O-Ring zu der vorbestimmten Position nahe dem Einlassabschnitt (12a) des Testbereichs (12) mitzunehmen;
- Ausführen einer ersten synchronisierten Drehung der Scheiben (l0, 11) in entgegengesetzter Richtung, um den O-Ring in der vorbestimmten Position abzufangen und den O-Ring in den Testbereich (12) in die erste gestörten Konfiguration zu ziehen und zu verriegeln;
- Aktivieren der Aufnahmemittel, um mindestens ein Bild des O-Rings in der ersten deformierten Konfiguration aufzunehmen;
- Ausführen einer weiteren synchronisierten Drehung in entgegengesetzter Richtung der Scheiben (10, 11), um den O-Ring aus dem Testbereich (12) durch den Auslassabschnitt (12b) auszustoßen.

16. Verfahren nach Anspruch 15, wobei das Verfahren den Schritt des Betätigens, nach der Aufnahme von Bildern in der ersten verformten Konfiguration und vor der Betätigung der weiteren synchronisierten Drehung, einer oder mehrerer synchronisierter Drehungen in derselben Richtung der Scheiben (10, 11) umfasst, von denen jede den O-Ring in einer entsprechenden verformten Konfiguration verformt und verriegelt, wobei das Verfahren weiter den Schritt des Betätigens der Aufnahmemittel umfasst, wenn jede verformte Konfiguration erreicht ist.

## Revendications

1. Montage d'essai (1) pour inspecter un joint torique (100), comprenant :
- un plan de support (30), sur lequel ledit joint torique repose ;
- un premier disque (10) et un second disque (11) coplanaires sur ledit plan de support (30) et agencés en position adjacente, étant configurés pour tourner sur ledit plan de support (30) autour d'axes (50, 51) respectifs ;
- des moyens d'acquisition d'image (40', 40") dudit joint torique agencés au moins sur un côté dudit plan de support (30) pour acquérir des images dudit joint torique,
**caractérisé en ce que** lesdits disques (10, 11) sont actionnés de manière réciproquement indépendante et définissent une région d'essai (12) au niveau de laquelle ledit joint torique est bloqué et déformé entre lesdits disques (10, 11), ledit plan de support (30) étant mobile et configuré pour porter ledit joint torique à la première position prédéterminée près d'une section d'entrée (12a) de ladite région d'essai (12),
dans lequel lesdits disques (10, 21) sont configurés pour effectuer une première rotation synchronisée dans un sens réciproquement opposé après quoi ledit joint torique est intercepté dans ladite première position prédéterminée, traîné et bloqué dans ladite région d'essai (12) dans une première configuration déformée, lesdits moyens d'acquisition d'image (40', 40") étant configurés pour acquérir au moins une première image dudit joint torique dans ladite première position déformée,
et dans lequel lesdits disques (10, 11) sont configurés pour effectuer au moins une rotation synchronisée supplémentaire dans un sens réciproquement opposé après quoi ledit joint torique est éjecté de ladite région d'essai (12) à travers une section de sortie (12b), sensiblement opposée à ladite section d'entrée (12a).

2. Montage (1) selon la revendication 1, dans lequel après ladite première rotation synchronisée dans un sens réciproquement opposé desdits disques (10, 11), le centre (C) dudit joint torique repose sur un plan défini par les axes de rotation (50, 51) desdits disques (10, 11).

3. Montage (1) selon la revendication 1 ou 2, dans lequel lesdits disques (10, 11) sont configurés pour effectuer, entre ladite première rotation et ladite seconde rotation synchronisées dans un sens opposé, au moins une rotation synchronisée dans le même sens après quoi ledit joint torique est déformé et bloqué dans ladite région d'essai (12) dans une seconde configuration déformée, différente de ladite première configuration déformée, lesdits moyens d'acquisition d'image (40', 40") étant configurés pour acquérir au moins une première image dudit joint torique dans ladite seconde configuration déformée.

4. Montage (1) selon la revendication 1 ou 2, dans lequel lesdits disques (10, 11) sont configurés pour effectuer, entre ladite première rotation et ladite seconde rotation synchronisées dans un sens opposé, une pluralité de rotations synchronisées dans le même sens après chacune desquelles ledit joint torique est déformé et bloqué dans ladite région d'essai (1) dans une configuration déformée, différente de ladite première configuration déformée, lesdits moyens d'acquisition d'image (40', 40") étant configurés pour acquérir au moins une première image dudit joint torique dans chaque configuration déformée.

5. Montage (1) selon l'une quelconque des revendications précédentes, dans lequel ledit premier montage comprend un premier moteur électrique (20a) et un second moteur électrique (20b) pour actionner ledit premier disque (10) et ledit second disque (11), respectivement.

6. Montage (1) selon l'une quelconque des revendications précédentes, dans lequel ledit montage comprend des premiers moyens de réglage pour régler la distance entre lesdits axes de rotation (50, 51) desdits disques (10, 11) en fonction de l'extension diamétrale dudit joint torique et de la déformation souhaitée.

7. Montage (1) selon la revendication 6, dans lequel ledit montage (1) comprend un cadre de support, qui inclut une barre transversale (80), un premier bras de support (72) et un second bras de support (74) pour supporter ledit premier disque (10) et ledit second disque (11), respectivement, lesdits bras (72, 74) étant reliés de manière coulissante sur ladite barre transversale pour régler ladite distance entre lesdits axes de rotation (50, 51) desdits disques (10, 11).

8. Montage (1) selon l'une quelconque des revendications précédentes, dans lequel ledit premier disque (10) et ledit second disque (11) comprennent une rainure circonférentielle (13, 14) adaptée pour loger et traîner ledit joint torique, respectivement.

9. Montage (1) selon l'une quelconque des revendications précédentes, dans lequel ledit plan de support (30) est fait d'un matériau transparent et dans lequel lesdits moyens d'acquisition comprennent une première caméra (41) et une seconde caméra (42) agencées en position symétrique par rapport audit plan de support (30).

10. Montage (1) selon la revendication 9, comprenant un premier illuminateur (42) agencé entre ledit plan de support (30) et ladite première caméra (41) et un second illuminateur (43) agencé entre ledit plan de support (30) et ladite seconde caméra (42).

11. Montage (1) selon l'une quelconque des revendications précédentes, dans lequel ledit montage (1) comprend une unité de commande (60) qui commande lesdits moteurs électriques (20a, 20b).

12. Montage (1) selon l'une quelconque des revendications 1 à 11, dans lequel ledit montage (1) comprend des moyens de traitement connectés auxdits moyens d'acquisition (40', 40") et à une unité de commande (60) qui commande lesdits moteurs électriques (20a, 20b) pour faire tourner lesdits disques (10, 11), lesdits moyens de traitement étant configurés pour vérifier la position du centre (C) dudit joint torique par rapport à une position idéale coïncidant avec un axe optique principal (500) défini par lesdits moyens d'acquisition d'image (40', 40"), ladite position réelle étant évaluée en analysant chaque image acquise par lesdits moyens d'acquisition d'image (40', 40").

13. Montage (1) selon la revendication 12, dans lequel lesdits moyens de traitement sont configurés pour comparer ladite position idéale afin d'établir une erreur de positionnement, lesdits moyens de traitement calculant les conditions de fonctionnement correspondantes pour la rotation synchronisée ultérieure desdits disques (10, 11) si ladite erreur de positionnement dépasse une valeur prédéterminée, ladite unité de commande (60) actionnant lesdits moteurs électriques (20a, 20b) en fonction desdites conditions de fonctionnement correspondantes.

14. Système d'inspection de joints toriques, **caractérisé en ce qu'**il comprend un montage d'essai (1) selon l'une quelconque des revendications 1 à 11.

15. Procédé d'inspection d'un joint torique au moyen d'un montage d'inspection (1) selon l'une quelconque des revendications 1 à 14, dans lequel ledit procédé comprend les étapes consistant à :
- agencer ledit joint torique sur ledit plan de support (30) ;
- agencer des moyens d'acquisition d'image du joint torique sur au moins un côté dudit plan de support (30) ;
- activer ledit plan de support (30) pour porter ledit joint torique vers ladite position prédéterminée près de ladite section d'entrée (12a) de ladite région d'essai (12) ;
- mettre en œuvre une première rotation synchronisée en sens opposé desdits disques (10, 11) pour intercepter ledit joint torique dans ladite position prédéterminée et pour traîner et bloquer ledit joint torique dans ladite région d'essai (12) dans ladite première configuration perturbée ;
- activer lesdits moyens d'acquisition pour acquérir au moins une image du joint torique dans ladite première configuration déformée ;
- mettre en œuvre une rotation synchronisée supplémentaire en sens opposé desdits disques (10, 11) pour expulser ledit joint torique de ladite région d'essai (12) à travers ladite section de sortie (12b).

16. Procédé selon la revendication 15, dans lequel ledit procédé comprend l'étape consistant à actionner, après l'acquisition d'images dans ladite première configuration déformée et avant l'actionnement de ladite rotation synchronisée supplémentaire, une ou plusieurs rotations synchronisées dans le même sens desdits disques (10, 11), chacune d'entre elles pour déformer et bloquer le joint torique dans une configuration déformée correspondante, ledit procédé comprenant en outre l'étape consistant à actionner lesdits moyens d'acquisition quand chaque configuration déformée est atteinte.
